# EUROPEAN PATENT SPECIFICATION

(11) **EP 0 974 669 B1**
(45) Date of publication and mention of the grant of the patent: **02.05.2007**
(21) Application number: 97903619.1
(22) Date of filing: 27.02.1997
(51) Int. Cl.: C12P 7/42, C12N 1/20, C12R 1/01, C12R 1/06

(54) **PROCESS FOR PREPARING ALPHA-HYDROXY ACIDS USING MICROORGANISM AND NOVEL MICROORGANISM**
PROZESS FÜR DIE HERSTELLUNG VON ALPHA-HYDROXYSÄUREN MIT HILFE EINES MIKROORGANISMUS UND EINES NEUEN MIKROORGANISMUS.
PROCESSUS DE PREPARATION D'ACIDES ALPHA-HYDROXY A L'AIDE D'UN MICRO-ORGANISME ET NOUVEAU MICRO-ORGANISME

(30) Priority: 29.02.1996 JP 6928896; 10.12.1996 JP 34665596
(43) Date of publication of application: 26.01.2000
(73) Proprietor: NIPPON SODA CO., LTD., Chiyoda-ku, Tokyo 100-8165 (JP)
(72) Inventor: KOBAYASHI, Yoichi, Odawara Research Center, Odawara-shi, Kanagawa 250-02 (JP); WATABE, Ken, Odawara Research Center, Odawara-shi, Kanagawa 250-02 (JP); OHIRA, Mahito, Odawara Research Center, Odawara-shi, Kanagawa 250-02 (JP); HAYAKAWA, Koichi, Odawara Research Center, Odawara-shi, Kanagawa 250-02 (JP)
(74) Representative: Schmitz, Jean-Marie
(86) International application number: PCT/JP1997/000578
(87) International publication number: WO 1997/032030

(56) References cited:
- EP-A- 0 610 049
- WO-A-96/09403
- JP-A- 4 040 898
- JP-A- 4 099 497
- JP-A- 5 192 189
- JP-A- 7 213 296
- JP-A- 63 222 696
- CHEMICAL ABSTRACTS, vol. 125, no. 13, 23 September 1996 Columbus, Ohio, US; abstract no. 165839, MATSUYAMA, AKIKAZU: "Microbial manufacture of.alpha.-hydroxy-4-methylthiobutyric acid" XP002104262 & JP 08 173175 A (DAICEL CHEM, JAPAN)
- CHEMICAL ABSTRACTS, vol. 126, no. 17, 28 April 1997 Columbus, Ohio, US; abstract no. 224356, NAKAMURA, TETSUJI: "Microbial manufacture of glycolic acid" XP002104263 & JP 09 028390 A (NITTO CHEMICAL INDUSTRY CO LTD, JAPAN)
- CHEMICAL ABSTRACTS, vol. 116, no. 23, 8 June 1992 Columbus, Ohio, US; abstract no. 233932, ENDO, RYUICHI: "Fermentative manufacture of.alpha.-hydroxyisobutyric acid" XP002104264 & JP 04 040897 A (NITTO CHEMICAL INDUSTRY CO., LTD., JAPAN)
- CHEMICAL ABSTRACTS, vol. 105, no. 7, 18 August 1986 Columbus, Ohio, US; abstract no. 59481, KAWAKAMI, KYOSHI ET AL: "Microbial production of.alpha.-hydroxy acid and its salts" XP002104265 & JP 61 056086 A (ASAHI CHEMICAL INDUSTRY CO., LTD., JAPAN)

## Description

The present invention concerns a method for producing α-hydroxy acids compounds according to claim 1, Arthrobacter NSSC 104 strain according to claim 7, as well as a method for producing compounds according to claim 8.

The present invention relates to a process to prepare α-hydroxy acid based on hydrolysis of hydroxy nitrile by means of using microorganisms and novel microorganisms. Among α-hydroxy acids, lactic acid is useful for foods, brewing and other industrial uses, while 2-hydroxy-4-methylthiobutyric acid is useful as a feed additive for livestock.

In the past, the following methods have been known as a method for preparing α-hydroxy acid [II] by using α-hydroxynitrile [I] as the starting material and by means of using microorganisms.
(1) A method for preparing lactic acid, glycolic acid, etc. by using a microorganism, such as *Bacillus* spp., *Bacterizium* spp., *Micrococcus* spp. and *Brevibacterium* spp., which is disclosed in Japanese Patent Publication No. Sho 58-15120.
(2) A method for preparing lactic acid, glycolic acid and 2-hydroxyisobutyric acid by using a microorganism belonging to *Corynebacterium* spp., which is disclosed in Japanese Patent Laid-open No. Sho 61-56086.
(3) A method for preparing lactic acid, 2-hydroxyisobutyric acid, 2-hydroxy-2-hydroxyphenyl propionic acid and mandelic acid by using a microorganism, such as *Pseudomonas* spp., *Arthrobacter* spp., *Aspergillus* spp., *Penicillium* spp.*, Cocryoboros* spp. and *Fusarium* spp., which is disclosed in Japanese Patent Laid-open No. Sho 63-222696.
(4) A method for preparing 2-hydroxy-3,3-dimethyl-4-butyrolactone by using a microorganism, such as *Arthrobacter* spp., *Aspergillus* spp., *Bacillus* spp., *Bacterizium* spp., *Brevibacterium* spp., *Cocryoboros* spp., *Corynebacterium* spp., *Micrococcus* spp., *Nocardia* spp., *Penicillium* spp., *Pseudomonas* spp. and *Fusarium* spp., which is disclosed in Japanese Patent Laid-open No. Sho 64-10996.
(5) A method for preparing 2-hydroxyisobutyric acid by using a microorganism, such as *Rhodococcus* spp., *Pseudomonas* spp., *Arthrobacter* spp. and *Brevibacterium* spp., which is disclosed in Japanese Patent Laid-open No. Hei 4-40897.
(6) A method for preparing α-hydroxy-4-methylthiobutyric acid by using a microorganism, such as *Caseobater* spp., *Pseudomonas* spp., *Alcaligenes* spp., *Corynebacterium* spp., *Brevibacterium* spp., *Nocardia* spp., *Rhodococcus* spp. and *Arthrobacter* spp., which is disclosed in Japanese Patent Laid-open No. Hei 4-40898.
(7) A method for preparing 4-methylthiobutyric acid by using a microorganism, such as *Alcaligenes* spp., *Rhodococcus* spp. and *Goldona* spp., which is disclosed in WO 96/09403.
(8) A method for preparing α-hydroxy-4-methylthiobutyric acid by using a microorganism, such as *Pantoea* spp., *Micrococcus* spp. and *Bacterizium* spp., which is disclosed in Japanese Patent Laid-open No. Hei 8-173175.

However, the methods for preparing α-hydroxy acid as mentioned above are not always recognized as satisfactory one which can produce and accumulate an objective substance at a high concentration. For example, in case of lactic acid, only 9.8% by weight accumulation thereof is recognized when one of *Corynebacterium* spp. is used as the microorganism (Japanese Patent Laid-open No. Sho 61-56086), only 10% by weight accumulation thereof is recognized when one of *Pseudomonas* spp. is used (Japanese Patent Laid-open No. Sho 63-222696) and only 0.15% by weight accumulation thereof is recognized when one of *Arthrobacter* spp. is used (Japanese Patent Laid-open No. Sho 63-222696). Whereas, the accumulation of α-hydroxyisobutyric acid by using one of *Pseudomonas* spp. is found to be as much as 0.8% by weight (Japanese Patent Laid-open No. Sho 63-222696), the accumulated amount of α-hydroxy-4-methylthiobutyric acid is found to be 188 mM (2.8% by weight) by using one of *Caseobater* spp. (Japanese Patent Laid-open No. Hei 4-40898), 55 mM (0.8% by weight) by using one of *Arthrobacter* spp. (Japanese Patent Laid-open No. Hei 4-40898) and 940 mM (14% by weight) by using one of *Alcaligenes* spp. (WO 96/09403), respectively.

As a reason for the low accumulation concentration of such products as described above, it is considered that some enzymatic activity relating thereto might be inhibited in the presence of cyanic acid (Agricultural Biological Chemistry, Vol.46, page 1165, 1982) which is generated in the partial dissociation of α-hydroxy nitrile in water together with the corresponding aldehyde or ketone (Chemical Reviews, Vol. 42, Page 189, 1948). Further, a possibility that the related-enzyme is inactivated in a short time with the dissociated-aldehyde has been also pointed out. As a solution to prevent such inactivation of the enzyme, a method to add either acidic sulfite ions or dithionite ions (Japanese Patent Laid-open No. Hei 5-192189) and a method to add either phosphite ions or hypophosphite ions (Japanese Patent Laid-open No. Hei 7-213296) have been proposed. However, the concentration of α-hydroxy acid produced and accumulated is not so high even by using such additives as described above.

Chemical abstracts, vol. 125, no. 13, 23 September 1996 (1996-09-23), Columbus, Ohio, US; abstract No. 165839, Matsuyama, Akikazu describes microbial manufacture of alpha-hydroxy-4-methylthiobutyric acid.

Chemical abstracts, vol. 126, no. 17, 28 April 1997 (1997-04-28), Columbus, Ohio, US; abstract no. 224356, Nakamura, Tetsuji relates to microbial manufacture of glycolic acid.

Chemical abstracts, vol. 116, no. 23, 8 June 1992 (1992-06-08), Columbus, Ohio, US; abstract no. 233932, Endo, Ryuichi concerns fermentative manufacture of alpha-hydroxyisobutyric acid.

Chemical abstracts, vol. 105, no. 7, 18 August 1986 (1986-08-18), Columbus, Ohio, US; abstract no. 59481, Kawakami, Kyoshi et al describes microbial production of alpha-hydroxy acid and its salts.

WO 96/09403 relates to a novel method for the enzymatic hydrolysis of 4-methylsthiobutyronitriles into racemic 4-methylthiobutyric acid using a nitrilase of alcaligenes faecalis, Rhodococcus sp. HT 29-7 or Gordona terrae.

EP 610 049 concerns a biological process for predominantly producing an optimally active α-hydroxycasrboxylic acid having a phenyl group directly from a racemic α-hydroxynitrile or a mixture of an aldehyde corresponding to the nitrile and prussic acid as a substrate is disclosed, comprising reacting a microorganism belonging to the genus Rhodococcus, Alcaligenes, Brevibacterium or Pseudomonas with the substrate in a neutral to basic acqueous medium. A desired optically active α-hydroxycarboxylic acid having a phenyl group can be obtained quantitatively at a high optical purity.

Database Biosis, abstract no. PREV197968047262 & Yagi & Minoda, Agriculture and Bio. Chem vol. 43, 1979, p. 571-576 describes lactic-acid production from 1 2 propanediol by jar culture and resting cells of arthrobacter-oxydans.

In general, when accumulated-concentration of a product remains low, it is well known to the specialists in the art that the installation for such manufacturing tend to be complex and large. Therefore, there has been difficulty in the efficiency to manufacture α-hydroxy acid in an industrial scale according to the methods as described above. The present invention is to provide a method to accumulate α-hydroxy acid at a high concentration level by means of using microorganisms and to efficiently produce α-hydroxy acid.

The method for producing α-hydroxy acids compounds is defined in claim 1, Arthrobacter NSSC 104 is defined in claim 7, the method for producing compounds is defined in claim 8.

The inventors of the present invention had made screening studies in order to find industrially-advantageous microorganisms which enzymatically convert α-hydroxy nitriles represented by a general formula [I]; RCH(OH)CN (wherein R represents a hydrogen atom, an optionally-substituted C1-C6 alkyl group, an optionally-substituted C2-C6 alkenyl group, an optionally-substituted C1-C6 alkoxy group, an optionally-substituted aryl group, an optionally-substituted aryloxy group, or an optionally-substituted heterocyclic group) to α-hydroxy acids represeted by a general formula [II]; RCH(OH)COOH (wherein R is as defined above), of which converting activity is resistant to the suppressing effect of α-hydroxy, nitrile [I] or α-hydroxy acid [II], and have durability to maintain such converting activity for a long time and capability to accumulate an α-hydroxy acid [II] to a high concentration level.

As a result, the inventors ultimately found such desired activity as described above in microorganisms which belong to *Variovorax* spp. and *Arthrobacter* spp. Furthermore, they have found that the enzymatic activity described above can be improved by adding a cyanide substance represented by a general formula [III]; Mm(CN)n (wherein M represents a hydrogen atom, ammonium or a metal ion, and m and n represent an integer 1, 2 or 3) into the reaction system to achieve the present invention.

Consequently, the present invention relates to a process for preparing α-hydroxy acid [II] characterized in that α-hydroxy acid [II] is produced, accumulated and harvested in an aqueous solvent in the presence of microorganisms having both concentration resistance and durable property to α-hydroxy nitrile [I] and/or α-hydroxy acid [II] in the process for preparing α-hydroxy acid [II] wherein the α-hydroxy nitrile [I] is hydrolyzed by the microbial reactions to thereby be converted to α-hydroxy acid [II], and a cyanide compound is added to the said reaction system.

The present invention is now further described in detail.

As the microorganisms to be used in the present invention, any one can be applied without particular limitation if the one can maintain its concentration resistance to either α-hydroxy nitriles or α-hydroxy acids to the extent required for achieving the object of the present invention and has durability to maintain the enzymatic activity for a long period. As the examples for such microorganisms, *Variovorax paradoxus* IAM 12374 strain and *Arthrobacter* NSSC 104 strain (FERM P-15424) are given. *Variovorax paradoxus* IAM 12374 strain is easily obtainable from Institute for Molecular Cell Biology, Tokyo University (IAM). Wheraes, *Arthrobacter* NSSC 104 strain has been newly isolated from the nature world by the inventors of the present invention and has been deposited with the following details.
Deposition No.: FERM BP-5829 (Transferred from Bikouken Microorganism Deposition No. P-15424)
Date of Deposition: Domestic deposition has been made on Feb. 6, 1996 and international deposition has been made on Feb. 20, 1997.
Place of Deposition: No. 1-3, Higashi 1-chome, Tsukuba-shi, Ibaragi, Japan
Organization for Deposition: Institute for Biotechnology and Industrial Technology, Industrial Technology Academy, Ministry of trade and Industries

Whereas, the microbiological property of *Arthrobacter* NSSC 104 strain is as follows.
Shape : Polymorphous bacillus
Gram's stain property : Positive
Rod-coccus cycle : Positive
Spores formation : Negative
Mobility : Negative
Diamino acid in Cell Wall : Lysine
Oxigen requirement : Aerobic
Oxidase formation : Negative
Catalase formation : Positive
DNA decomposition : Positive
Liquefaction of Gelatin : Positive
Starch decomposition : Positive
Casein decomposition : Positive
Vitamins requirement : Negative
Glycolyl test : Negative (Acetyl-type)
Quinone type : MK-9 (H2)
Sugar composition of Cell Wall : Galactose +
   Glucose +

After referring the microbiological properties of the NSSC 104 strain according to Bergey's Manual of Systematic Bacteriology (1986), the strain was identified as a bacterial strain belonging to *Arthrobacter* spp. The strain has been deposited with the deposition No. mentioned above in Institute for Biotechnology and Industrial Technology, Industrial Technology Academy, Ministry of Trade and Industries

Now, the embodiments for carrying out the present invention are described herein below.

Cultivation of the microorganisms used in the present invention is carried out on an ordinary medium wherein enzyme-induction substance, carbon source being utilizable by the microorganisms, nitrogen source, inorganic ions and organic nutrients, if required, are contained. As the examples for the enzyme induction substance usable in the present invention, nitrile compounds including isobutyronitrile and the like and cyclic amide compounds including ε-caprolactam and the like are given. As the carbon source, carbohydrates including glucose and the like, alcohols including ethanol and the like, organic acids and so on are used if required. As the nitrogen source, amino acids, nitrates, ammonium salts and the like are used. As the inorganic ions, phosphate ions, potassium ions, magnesium ions, sulfate ions, ferric ions and the like are used depending upon the requirement. As the organic nutrients, vitamines, amino acids, etc. and corn steep liquer containing the same, yeast extracts, polypeptone, bouillon extracts and the like are used, if required. The cultivation is carried out by properly maintaining the medium condition, PH to a range from 6 to 9, temperature to a range of from 25 to 37 °C and under an aerobic condition.

The hydrolysis reaction caused by microorganisms according to the present invention is proceeded by collecting the cultivated microorganism cells as described above, preparing processed-microorganism cells, such as immobilized cells, crude enzymes and immobilized enzymes, and contacting the processed-microorganisms to α-hydroxy nitrile [I] in an aqueous solvent. When immobilizing microorganism cells or enzymes, a normal immobilization method, such as carrier binding method and entrapping method, can be applied. When preparing crude enzymes, enzyme purification methods customarily employed, such as ammonium sulfate salting out and chromatography, can be applied after crushing microorganism cells by using ultrasonic waves, high-pressure homogenizer or the like. The microorganism cells is used for the hydrolysis reaction at a dose of 0.01-10% by weight on dry weight basis, and the cells can be repeatedly used by recovering them by any means of filtration, centrifugation and ultrafiltration membrane concentration method after the completion of the reaction. As the aqueous solvent, either water or aqueous solution containing minerals such as buffer and an organic solvent can be used, and said aqueous solution may be separated into two layers.

R contained in α-hydroxy nitrile represented by the general formula [I]; RCH(OH)CN, in the present invention represents a hydrogen atom, an optionally-substituted C1-C6 alkyl group, an optionally-substituted C2-C6 alkenyl group, an optionally-substituted C1-C6 alkoxyl group, an optionally-substituted aryl group, an optionally-substituted aryloxy group or an optaionally-substituted heterocyclic group.

More specifically, R can be a hydrogen atom, a C1-C6 alkyl, such as methyl, ethyl, propyl, isopropyl, butyl, sec-butyl, t-butyl, pentyl and hexyl,
a C1-C6 alkylthio C1-C6 alkyl, such as methylthiomethyl, 1-methylthioethyl, 2-methylthioethyl, 1-methylthiopropyl, 2-methylthiopropyl, 3-methylthiopropyl, 1-methylthiobutyl, 2-methylthiobutyl, 3-methylthiobutyl, 4-methylthiobutyl, 6-methylthiohexyl, ethylthiomethyl, 1-ethylthioethyl, 2-ethylthioethyl, 1-ethylthiopropyl, 2-ethylthiopropyl, 3-ethylthiopropyl, 1-ethylthiobutyl, 2-ethylthiobutyl, 3-ethylthiobutyl, 4-ethylthiobutyl, propylthiomethyl, 1-propylthioethyl, 2-propylthioethyl, 1-propylthiopropyl, 2-propylthiopropyl, 3-propylthiopropyl, 1-methylthioisopropyl, 1-ethylisopropyl, 1-propylthiobutyl, 2-propylthiobutyl, 3-propylthiobutyl, 4-propylthiobutyl, propylthiomethyl, 1-propylthioethyl, 2-propylthioethyl, 1-isopropylthiopropyl, 2-isopropylthiopropyl, 3-isopropylthiopropyl, 1-isopropylthiobutyl, 2-isopropylthiobutyl, 3-isopropylthiobutyl and 4-isopropylthiobutyl,
a hydroxy C1-C6 alkyl, such as hydroxymethyl, 1-hydroxyethyl, 2-hydroxyethyl, 1-hydroxypropyl, 2-hydroxypropyl, 3-hydroxypropyl, 1-hydroxybutyl, 1-hydroxyisopropyl, 2-hydroxybutyl and 3-hydroxybutyl,
a carboxy C1-C6 alkyl, such as carboxymethyl, 2-carboxyethyl, 1-carboxyethyl, 3-carboxypropyl, 2-carboxypropyl and 1-carboxypropyl,
a carbamoyl C1-C6 alkyl, such as carbamoylmethyl, 1-carbamoylethyl, 2-carbamoylethyl, 1-carbamoylpropyl, 2-carbamoylpropyl and 3-carbamoylpropyl,
a mercapto C1-C6 alkyl, such as mercaptomethyl, 1-mercaptoethyl, 2-mercaptoethyl, 1-mercaptopropyl, 2-mercaptopropyl and 3-mercaptopropyl,
a carbamidino C1-C6 alkyl, such as carbamidinomethyl, 1-carbamidinoethyl, 2-carbamidinoethyl, 1-carbamidinopropyl, 2-carbamidinopropyl and 3-carbamidinopropyl,
an optionally-substituted C1-C6 aralkyl, such as benzyl, 2-chlorobenzyl, 4-methylbenzyl, 4-methoxybenzyl, 3-nitrobenzyl, 4-hydroxybenzyl, α-methylbenzyl and α, α-dimethylbenzyl,
a C1-C6 alkyl substituted with a heterocyclic ring, such as 3-indolylmethyl, 2-indolylmethyl, 2-(3-indolyl)ethyl, 1-(3-indolyl)ethyl, 2-indolylmethyl, 2-(2-indolyl)ethyl, 1-(2-indolyl)ethyl, 4-imidazolylmethyl, 2-imidazolylmethyl, 1-(4-imidazolyl)ethyl and 2-(4-imidazolyl)ethyl,
an optionally-substituted C2-C6 alkenyl, such as vinyl, propenyl, isopropenyl, allyl, 1-chloroallyl, 2-chloroallyl and crothyl,
an optionally-substituted C1-C6 alkoxyl, such as methoxy, ethoxy, propoxy and trifluoromethoxy,
an optionally-substituted aryl, such as phenyl, 2-chlorophenyl, p-tolyl, 3-nitrophenyl, 4-cyanophenyl, α-naphthyl and β-naphthyl,
an optionally-substituted aryloxy, such as phenyloxy, 2-chlorophenyloxy, p-tolyloxy, 3-nitrophenyloxy, α-naphthyloxy and β-naphthyloxy, or,
a 3-7 membered heterocyclic group which contains at least one atom selected from a group consisting of nitrogen atom, oxygen atom and sulfur atom as hetero atom, such as 2-pyridyl, 3-pyridyl, 4-pyridyl, 5-chloro-3-pyridyl, 2-thienyl, 3-thienyl, 2-pyrrolyl, 3-pyrrolyl, 2-furyl and 3-furyl.

As more specific examples for the α-hydroxy nitrile [I], lactonitrile, mandelonitrile, 2-hydroxy-4-methylthiobutyronitrile and the like can be given.

The The α-hydroxy nitrile [I] is used in the reaction at a concentration of 0.1-50% by weight and may be added further during the process of the reaction when required. pH of the reaction solution shall be kept in a range of from 5 to 11 by using either appropriate buffers or acid and alkali. The reaction is preferably proceeded at a temperature of from 4 to 50°C, more preferably from 20 to 40 °C.

As the examples for the cyanide compounds used in the present invention and represented by a general formula [III], hydrogen cyanide, sodium cyanide, potassium cyanide, calcium cyanide, magnesium cyanide, tarium cyanide, ammonium cyanide and the like are given. The cyanide compound is normally used in the reaction at a concentration of from 0.4 to 1000 mM, preferably from 4 to 500 mM and may be added further during the reaction when required.

Consequently, α-hydroxy acid [II] corresponding to α-hydroxy nitrile [I] which is added for a period of from 6 to 120 hours is accumulated as the ammonium salt thereof at a concentration of 15% by weight or more. The microorganism cells used in the reaction can be repeatedly used for the hydrolysis reaction without substantial loss of enzymatic activity.

The product obtained can be isolated and purified according to a cutomarily-employed method, such as concentration and extraction, and the product can be separated from ammonium by means of extraction with an organic solvent, thermal decomposition or the like, if required. As the examples for the product, that is α-hydroxy acid represented by the formula [II], lactic acid, mandelic acid. 2-hydroxy-4-methylthiobutyric acid and the like are given.

### Best Mode for Carrying Out the Invention

The present invention is further explained in detail with referring the following examples.

### (Example 1)

A medium in an amount of 2 ml containing 0.3% buillon, 0.5% pepton and 0.5% sodium chloride was placed in a test tube, and the other medium in an amount of 20 ml having a composition as mentioned below was placed in a 100 ml triangular-shaped flask with buffles, then both media were sterilized for 15 minutes at 121 °C. respectively. One loop amount of IAM 12374 strain of *Variovorax paradoxus* was inoculated into the test tube containing 2 ml medium and the strain was cultivated under continuous shaking condition for over night at 30 °C, then 0.2ml of the medium was transferred to the triangular-shaped flask with buffles. The transferred-medium was further cultivated at 30°C for 3 days. The cultivated-medium was then subjected to centrifugation process, and the microorganism cells obtained was washed with saline. The microorganism cells was then suspended into 0.1 M phosphate buffer solution (pH 7.5) to prepare 0.1 % by weight solution on dry weight basis. Then, 2-hydroxy-4-methylthiobutyronitrile was added into the phosphate buffer solution to prepare 160 mM solution thereof as final concentration and hydrolysis reaction of the solution was proceeded at 30 °C with mild shaking. After the addition, the same amount of 2-hydroxy-4-methylthiobutyronitrile was further added 9 times to the buffer solution at an interval of 12 hours to carry out the reaction for total duration of 120 hours. After the completion of the reaction, the reacted-solution was subjected to a centrifugation process to remove the microorganism cells and the concentration of 2-hydroxy-4-methylthiobutyric acid contained in the supernatant was determined by using high performance liquid chromatography (Column: TSK gel ODS-80TM, Carrier: acetonitrile/water/trifluoroacetic acid=20/80/0.1). The accumulation of 2-hydroxy-4-methylthiobutylate ammonium at a level of 25% by weight was thereby confirmed. The yield was found to be 98%.

| | |
|---|---|
| Yeast Extract | 0.5% |
| Glycerol | 0.5% |
| Monopotassium phosphate | 0.1% |
| Dipotassium phosphate | 0.1% |
| NaCl | 0.02% |
| Magnesium sulfate 7 hydrate | 0.02% |
| ε-caprolactam | 0.5% |
| pH | 7.2 (Adjusted with 2N sodium hydroxide) |

### (Example 2)

A medium in an amount of 2 ml containing 0.3% buillon, 0.5% pepton and 0.5% sodium chloride was placed in a test tube, and the other medium in an amount of 20 ml having a composition as mentioned below was placed in a 100 ml triangular-shaped flask with buffles, then both media were sterilized for 15 minutes at 121 °C, respectively. One loop amount of NSSC 104 strain of *Arthrobacter* spp. was inoculated into the test tube containing 2 ml medium and the strain was cultivated under continuous shaking condition for a night at 30°C, then 0.2ml of the medium was transferred to the triangular-shaped flask with a buffle. The transferred-medium was further cultivated under continuous shaking condition at 30°C for 5 days. The cultivated-medium was then subjected to centrifugation process, and the microorganism cells obtained was washed with saline. The microorganism cells was then suspended into 0.1 M phosphate buffer solution (pH 7.5) to prepare 0.6 % by weight solution thereof on dry weight basis. Then, lactonitrile was added into the phosphate buffer solution to thereby prepare 124 mM buffer solution as its final concentration and the buffer solution was then subjected to hydrolysis reaction at 30 °C with mild shaking. After the addition, the same amount of lactonitrile was further added 20 times to the buffer solution at an interval of 5 hours to carry out the reaction for total duration of 100 hours. After the completion of the reaction, the reacted-solution was subjected to a centrifugation process to remove the microorganism cells and the concentration of lactic acid contained in the supernatant was determined by using high performance liquid chromatography (Column: TSK gel ODS-80TM, Carrier: acetonitrile/water/trifluoroacetic acid = 5/95/0.1). The accumulation of lactate ammonium salt at a level of 23% by weight was thereby determined. The yield was found to be 93%.
Corn steep liquer : 1.0% (Separately sterilized)
Sucrose : 1.0% (Separately sterilized)
Monopotassium phosphate : 0.1%
Dipotassium phosphate : 0.1 %
NaCl : 0.02%
Magnesium sulfate 7 hydrates : 0.02%
Ferrous sulfate : 0.001% (Separately sterilized)
ε-caprolactam : 0.5%
pH : 7.2 (Adjusted with 2N sodium hydroxide)

### (Example 3)

According to the same procedure described in the example 2, NSSC 104 strain of *Arthrobacter* spp. was suspended in 0.1M phosphate buffer solution (pH 7.5) to prepare 4% by weight solution thereof on dry weight basis. Then, 2-hydroxy-4-methylthiobutyronitrile was added to the buffer solution to prepare 200 mM solution thereof as its final concentration, and the buffer solution was subjected to hydrolysis reaction at 30°C with mild shaking. After the addition, the same amount of 2-hydroxy-4-methylthiobutyronitrile was repeatedly added to the hydrolyzed solution 7 times at an interval of 1 hour and further added 8 times at an interval of 1.5 hours to subject the solution to the reaction for total duration of 19 hours. After the completion of the reaction, the reacted-solution was centrifuged to remove microorganism cells, and the concentration of 2-hydroxy-4-methylthiobutyric acid contained in the supernatant was determined accordig to the same procedure as described in the example 1. The accumulation of 2-hydroxy-4-methylthiobutyrate ammonium salt at a level of 49% by weight was determined. The yield was found to be 96%.

### (Example 4)

According to the same procedure described in the example 2, NSSC 104 strain of *Arthrobacter* spp. was suspended in distillated water to prepare 3.2% by weight solution thereof on dry weight basis. Then, 2-hydroxy-4-methylthiobutyronitrile was continuously added to the suspension at a rate of 0.46 g/hr per 1g microorganism cells on dry weight basis. The suspension was then subjected to hydrolysis reaction at 30 °C for 20 hours while adjusting the pH with 0.5M aqueous ammonium solution to a range of from 7.4-7.6. After the completion of the reaction, the reacted-suspension was centrifuged to remove the microorganism cells. The microorganism cells collected was then washed 3 times with using 40-folds weight amount of distilled water, and the washed-cells were suspended again in distilled water in the same amount used for the first washing and was used for the second reaction. According to the same procedure for the first washing, all of the second reaction, recovery of microorganism cells and washing of microorganism cells were proceeded. Afetr repeating such reaction process as described above 10 times, the concentration of 2-hydroxy-4-methylthiobutyric acid contained In the supernatant was determined for each replications according to the method as described in the example 1. The results are shown in the following table.

| Replication of Reaction | 2-hydroxy-4-methylthiobutyrate ammonium salt | Yield (%) |
|---|---|---|
| | (% by weight) | |
| 1 | 36.1 | 96 |
| 3 | 36.3 | 97 |
| 5 | 36.4 | 97 |
| 7 | 36.4 | 97 |
| 10 | 36.0 | 96 |

### (Example 5)

According to the same procedure described in the example 2. NSSC 104 strain of *Arthrobacter* spp. was suspended in 0.1M aqueous solution of sodium cyanide to prepare 5% by weight solution thereof on dry weight basis. Then, lactonitrile was continuously added to the solution and was then subjected to hydrolysis reaction at 30 °C for 10 hours while adjusting the pH by using a pH controller to a range of from 7.4-7.6. After the completion of the reaction, the reacted-solution was centrifuged to remove microorganism cells, and the concentration of lactic acid contained in the supernatant was determined according to the same procedure described in the example 2. Another reaction where the addition of sodium cyanide was omitted was also checked for the comparison. The results are shown in the following table.

| | Amount of lactate ammonium salt produced (% by weight) |
|---|---|
| No addition | 20.5 |
| 0.1 M NaCN | 40.0 |

### (Example 6)

According to the same procedure described in the example 2, NSSC 104 strain of *Arthrobacter* spp. was suspended in 0.1M aqueous solution of potassium cyanide to prepare 5% by weight solution thereof on dry weight basis. Then, 2-hydroxy-4-methylthiobutyronitrile was continuously added to the solution and was then subjected to hydrolysis reaction at 30°C for 10 hours while adjusting the pH by using a pH controller to a range of from 7.4-7.6. After the completion of the reaction, the reacted-solution was centrifuged to remove microorganism cells, and the concentration of 2-hydroxy-4-methylthiobutyric acid contained in the supernatant was determined according to the same procedure described in the example 1. Another reaction where the addition of potassium cyanide was omitted was also checked for the comparison. The results are shown in the following table.

| | Amount of 2-hydroxy-4-methylthiobutyrate ammonium salt |
|---|---|
| | (% by weight) |
| No addition | 26.6 |
| 0.1M KCN | 41.7 |

### (Example 7)

According to the same procedure described in the example 2, NSSC 104 strain of *Arthrobacter* spp. was suspended in 40 mM aqueous solution of hydrogen cyanide to prepare 5% by weight solution thereof on dry weight basis. Then, 2-hydroxy-4-methylthiobutyronitrile was continuously added to the solution and was then subjected to hydrolysis reaction at 30°C for 10 hours while adjusting the pH by using a pH controller to a range of from 7.4-7.6. After the completion of the reaction, the reacted-solution was centrifuged to remove microorganism cells, and the concentration of 2-hydroxy-4-methylthiobutyric acid contained in the supernatant was determined according to the same procedure described in the example 1. Another reaction where the addition of hydrogen cyanide was omitted was also checked for the comparison. The results are shown in the following table.

| | Amount of 2-hydroxy-4-methylthiobutyrate ammonium salt produced (% by weight) |
|---|---|
| No addition | 27.2 |
| 40 mM HCN | 44.5 |

### (Example for Comparison)

The cultivation and the catalytic reaction of *Arthrobacter* NSSC 104 strain was carried out according to the cultivation method and the hydrolysis method described in Japanese Patent Laid-open No. Hei 4-40898.

### (1) Medium (Unit: W/V)

Glycerol 2%
Yeast extract 0.3%
Monopotassium phosphate 0.68%
Dipotassium phosphate 0.71%
Sodium sulfate 0.28%
Magnesium chloride 0.04%
Calcium chloride 0.004%
Manganese sulfate 0.0004%
Ferrous chloride 0.00006%
Zinc sulfate 0.00005%
Agar 1.8%
α-chloropropionitrile 0.05%
pH 7.5

### (2) Cultivating condition

One loop amount of the microorganism cells was taken out of the medium and was inoculated onto agar plate medium, and then cultivated at 30°C for 48 hours under aerobic condition.

### (3) Hydrolysis reaction

The microorganism cells was collected from the agar plate medium and then washed 3 times with 0.05M phosphate buffer (pH 7.5) by means of centrifugation.

The cells precipitated was re-suspended in 0.05M phosphate buffer in an amount of 1.5 ml so as to adjust the value of the OD 630 to 25, added with 100 mM 2-hydroxy-4-methylthiobutyronitrile as its final concentration and then allowed to a reaction at 25 °C for 20 hours under shaking condition. After the completion of the reaction, the reacted-solution was centrifuged to remove the microorganism cells, and the concentration of 2-hydroxy-4-methylthiobutyric acid contained in the supernatant was determined by using high performance liquid chromatography (Column: TSK gel ODS-80TM, Carrier: acetonitrile/water/tri fluoroacetic acid = 20/80/0.1). The concentration of 2-hydroxy-4-methylthiobutyric acid was 0.01 mM.

It is described in Japanese Patent Laid-open No. Hei 4-40898 that *Arthrobacter* HR4 strain achieved the accumulation of 2-hydroxy-4-methylthiobutyric acid at a level of 5 mM according to the cultivation method and the hydrolysis method as described above. Therefore, it is clearly indicated that *Arthrobacter* NSSC 104 strain is obviously different species from *Arthrobacter* HR4 strain.

### (Advantageous Effect of the Invention)

According to the present invention, the production of α-hydroxy acid [II] in an efficient manner can be achieved by means of utilizing enzymatic reaction of microorganisms which can be repeatedly used, having both concentration resistance and durability against α-hydroxy nitrile [I] and/or α-hydroxy acid [II] and allows the accumulation of α-hydroxy acid [II] at a high concentration level. In addition, by adding a cyanide compound to the reaction system, the production of α-hydroxy acid [II] in more efficient manner can be achieved.

### Industrial Use of the Invention

The present invention is related to a process for producing α-hydroxy acid [II] by using α-hydroxy nitrile as the starting material in an industrial scale and by utilizing the enzymatic reaction of microorganisms which have both concentration resistance and durable property to α-hydroxy nitrile [I] and/or α-hydroxy acid [II], which provides great significance in the related-industry.

## Claims

1. A method for producing compounds represented by a general formula [II]; RCH(OH)COOH, in which R represents a hydrogen atom, an optionally-substituted C1-C6 alkyl group, an optionally-substituted C2-C6 alkenyl group, an optionally-substituted C1-C6 alkoxy group, an optionally-substituted aryl group, an optionally-substituted aryloxy group or an optionally-substituted heterocyclic group, **characterized in that** the compounds represented by the general formula [II]; RCH(OH)COOH, in which R is as defined above, are produced and accumulated in an aqueous solvent in the presence of microorganism having both concentration resistance and durable property to compounds represented by a general formula [I]; RCH(OH)CN, in which R is as defined above, and/or the compounds represented by the general formula [II]; RCH(OH)COOH, in which R is as defined above, in the process for converting a compound represented by the general formula [I]; RCH(OH)CN, in which R is as defined above, to the compounds represented by the general formula [II]; RCH(OH)COOH, in which R is as defined above, by utilizing a hydrolytic reaction provided by the enzymatic activity of the microorganism, the microorganism being Variovorax paradoxus or Arthrobacter NSSC 104 strain, deposited as FERM BP-5829.

2. A method for producing compounds represented by the general formula [II]; RCH(OH)COOH according to the claim 1, **characterized in that** R is a hydrogen atom, an optionally-substituted C1-C6 alkyl group or an optionally-substituted phenyl.

3. A method for producing compounds represented by the general formula [II]; RCH(OH)COOH, according to claim 1 or 2, **characterized in that** R is a hydrogen atom, a C1-C6 alkylthio alkyl group, a C1-C6 hydroxy alkyl group or phenyl.

4. A method for producing compounds represented by the general formula [II]; RCH(OH)COOH, according to claim 1, **characterized in that** the compound represented by the general formula [I]; RCH(OH)CN, is one selected from the group consisting of lactonitrile, mandelonitrile and 2-hydroxy-4-methylthiobutyronitrile.

5. A method for producing compounds represented by the general formula [II]; RCH(OH)COOH, according to any one of claims 1 through 4, **characterized in that** the microorganism is Variovorax paradoxus.

6. A method for producing compounds represented by the general formula [II]; RCH(OH)COOH, according to any one of claims 1 through 4, **characterized in that** the microorganism is Arthrobacter NSSC 104 strain, FERM BP-5829.

7. Arthrobacter NSSC 104 strain, FERM BP-5829, which belongs to Arthrobacter spp., **characterized by** having concentration resistance and durable property to compounds represented by a general formula [I]; RCH(OH)CN, in which R represents a hydrogen atom, an optionally-substituted C1-C6 alkyl group, an optionally-substituted C2-C6 alkenyl group, an optionally-substituted C1-C6 alkoxy group, an optionally-substituted aryl group, an optionally-substituted aryloxy group or an optionally-substituted heterocyclic group, and/or compounds represented by a general formula [II]; RCH(OH)COOH, in which R is as defined in Claim 1, and having a capability to convert a compound represented by the general formula [I]; RCH(OH)CN, to a compound represented by the general formula [II]; RCH(OH)COOH.

8. A method for producing compounds represented by a general formula [II]; RCH(OH)COOH, in which R represents a hydrogen atom, an optionally-substituted C1-C6 alkyl group, an optionally-substituted C2-C6 alkenyl group, an optionally-substituted C1-C6 alkoxy group, an optionally-substituted aryl group, an optionally-substituted aryloxy group or an optionally-substituted heterocyclic group, **characterized in that**, in the method for producing the compounds represented by the general formula [II]; RCH(OH)COOH, in which R is as defined in Claim 1, based on hydrolysis of a compound represented by a general formula [I]; RCH(OH)CN, in which R is as defined above, by means of utilizing the enzymatic activity of a microorganism which is Variovorax paradoxus or Arthrobacter NSSC 104 strain, FERM BP-5829 the reaction is carried out in the presence of a cyanide compound represented by a general formula [III]; Mm(CN)n, in which M represents a hydrogen atom, ammonium or a metal ion, and m and n each independently represents an integer of 1, 2 or 3.

9. A method for producing compounds represented by the general formula [II]; RCH(OH)COOH, according to claim 8, **characterized in that** R is hydrogen, an optionally-substiuted C1-C6 alkyl group or an optionally-substituted phenyl.

10. A method for producing compounds represented by the general formula [II]; RCH(OH)COOH, according to claim 8 or 9, **characterized in that** R is hydrogen, an C1-C6 alkylthioalkyl group, C1-C6 hydroxy alkyl group or phenyl.

11. A method for producing compounds represented by the general formula [II]; RCH(OH)COOH, according to claim 8, **characterized in that** the compound represented by the general formula [I]; RCH(OH)CN, in which R is as defined in Claim 1, is one selected from the group consisting of lactonitrile, mandelonitrile and 2-hydroxy-4-methylthiobutyronitrile.

12. A method for producing compounds represented by the general formula [II]; RCH(OH)COOH, according to claim 8, **characterized in that** the microorganism is Variovorax paradoxus.

13. A method for producing compounds represented by the general formula [II]; RCH(OH)COOH, according to claim 8, **characterized in that** the microorganism is Arthrobacter NSSC 104 strain, FERM-BP-5829.

## Patentansprüche

1. Verfahren zur Herstellung von Verbindungen, die dargestellt werden durch eine allgemeine Formel [II] ; RCH(OH)COOH, worin R ein Wasserstoffatom, eine gegebenenfalls substituierte C1-C6-Alkylgruppe, eine gegebenenfalls substituierte C2-C6-Alkenylgruppe, eine gegebenenfalls substituierte C1-C6-Alkoxygruppe, eine gegebenenfalls substituierte Arylgruppe, eine gegebenenfalls substituierte Aryloxygruppe oder eine gegebenenfalls substituierte heterozyklische Gruppe darstellt, **dadurch gekennzeichnet, dass** die Verbindungen, dargestellt durch die allgemeine Formel [II] ; RCH(OH)COOH, worin R ist wie oben definiert, in einem wässrigen Lösungsmittel in Gegenwart eines Mikroorganismus produziert und akkumuliert werden, der sowohl eine Konzentrationsresistenz- als auch Haltbarkeitseigenschaft aufweist gegenüber Verbindungen, die dargestellt werden durch eine allgemeine Formel [I] ; RCH(OH)CN, worin R ist wie oben definiert, und/oder den Verbindungen, die dargestellt werden durch die allgemeine Formel [II] ; RCH(OH)COOH, worin R ist wie oben definiert, in dem Verfahren zum Umwandeln einer Verbindung, die dargestellt wird durch die allgemeine Formel [I] ; RCH(OH)CN, worin R ist wie oben definiert, zu den Verbindungen, dargestellt durch die allgemeine Formel [II] ; RCH(OH)COOH, worin R ist wie oben definiert, durch Anwendung einer hydrolytischen Reaktion, die durch die enzymatische Aktivität des Mikroorganismus bereitgestellt wird, wobei der Mikroorganismus Variovorax paradoxus oder Arthrobacter Stamm NSSC 104, hinterlegt als FERM BP-5829, ist.

2. Verfahren zur Herstellung von Verbindungen, dargestellt durch die allgemeine Formel [II] ; RCH(OH)COOH, gemäß dem Anspruch 1, **dadurch gekennzeichnet, dass** R ein Wasserstoffatom, eine gegebenenfalls substituierte C1-C6-Alkylgruppe oder ein gegebenenfalls substituiertes Phenyl ist.

3. Verfahren zur Herstellung von Verbindungen, dargestellt durch die allgemeine Formel [II] ; RCH(OH)COOH, gemäß Anspruch 1 oder 2, **dadurch gekennzeichnet, dass** R ein Wasserstoffatom, eine C1-C6-Alkylthioalkylgruppe, eine C1-C6-Hydroxyalkylgruppe oder Phenyl ist.

4. Verfahren zur Herstellung von Verbindungen, dargestellt durch die allgemeine Formel [II] ; RCH(OH)COOH, gemäß Anspruch 1, **dadurch gekennzeichnet, dass** die Verbindung, dargestellt durch die allgemeine Formel [I] ; RCH(OH)CN, eine aus der aus Lactonitril, Mandelonitril und 2-Hydroxy-4-methylthiobutyronitril bestehenden Gruppe gewählte Verbindung ist.

5. Verfahren zur Herstellung von Verbindungen, dargestellt durch die allgemeine Formel [II] ; RCH(OH)COOH, gemäß einem der Ansprüche 1 bis einschließlich 4, **dadurch gekennzeichnet, dass** der Mikroorganismus Variovorax paradoxus ist.

6. Verfahren zur Herstellung von Verbindungen, dargestellt durch die allgemeine Formel [II] ; RCH(OH)COOH, gemäß einem der Ansprüche 1 bis einschließlich 4, **dadurch gekennzeichnet, dass** der Mikroorganismus Arthrobacter Stamm NSSC 104, FERM BP-5829 ist.

7. Arthrobacter Stamm NSSC 104, FERM BP-5829, der zur Spezies Arthrobacter gehört, **dadurch gekennzeichnet, dass** er eine Konzentrationsresistenz- und Haltbarkeitseigenschaft gegenüber Verbindungen hat, die dargestellt sind durch eine allgemeine Formel [I] ; RCH(OH)CN, worin R ein Wasserstoffatom, eine gegebenenfalls substituierte C1-C6-Alkylgruppe, eine gegebenenfalls substituierte C2-C6-Alkenylgruppe, eine gegebenenfalls substituierte C1-C6-Alkoxygruppe, eine gegebenenfalls substituierte Arylgruppe, eine gegebenenfalls substituierte Aryloxygruppe oder eine gegebenenfalls substituierte heterozyklische Gruppe darstellt, und/oder Verbindungen, dargestellt durch eine allgemeine Formel [II] ; RCH(OH)COOH, worin R ist wie in Anspruch 1 definiert und eine Fähigkeit hat, eine Verbindung, dargestellt durch die allgemeine Formel [I] ; RCH(OH)CN, in eine Verbindung, dargestellt durch die allgemeine Formel [II] ; RCH(OH)COOH umzuwandeln.

8. Verfahren zur Herstellung von Verbindungen, dargestellt durch eine allgemeine Formel [II] ; RCH(OH)COOH, worin R ein Wasserstoffatom, eine gegebenenfalls substituierte C1-C6-Alkylgruppe, eine gegebenenfalls substituierte C2-C6-Alkenylgruppe, eine gegebenenfalls substituierte C1-C6-Alkoxygruppe, eine gegebenenfalls substituierte Arylgruppe, eine gegebenenfalls substituierte Aryloxygruppe oder eine gegebenenfalls substituierte heterozyklische Gruppe darstellt, **dadurch gekennzeichnet, dass** in dem Verfahren zur Herstellung der Verbindungen, dargestellt durch die allgemeine Formel [II] ; RCH(OH)COOH, worin R ist wie in Anspruch 1 definiert, auf Basis von Hydrolyse einer Verbindung, dargestellt durch eine allgemeine Formel [I] ; RCH(OH)CN, worin R wie oben definiert ist, mittels der Nutzung der enzymatischen Aktivität eines Mikroorganismus, welcher Variovorax paradoxus oder Arthrobacter Stamm NSSC 104, FERM BP-5829 ist, die Reaktion in Gegenwart einer Cyanidverbindung durchgeführt wird, dargestellt durch eine allgemeine Formel [III] ; Mm(CN)n, worin M ein Wasserstoffatom, Ammonium oder ein Metallion darstellt und m und n jedes unabhängig eine ganze Zahl von 1, 2 oder 3 darstellt.

9. Verfahren zur Herstellung von Verbindungen, dargestellt durch die allgemeine Formel [II] ; RCH(OH)COOH, gemäß Anspruch 8, **dadurch gekennzeichnet, dass** R Wasserstoff, eine gegebenenfalls substituierte C1-C6-Alkylgruppe oder ein gegebenenfalls substituiertes Phenyl ist.

10. Verfahren zur Herstellung von Verbindungen, dargestellt durch die allgemeine Formel [II] ; RCH(OH)COOH, gemäß Anspruch 8 oder 9, **dadurch gekennzeichnet, dass** R Wasserstoff, eine C1-C6-Alkylthioalkylgruppe, C1-C6-Hydroxyalkylgruppe oder Phenyl ist.

11. Verfahren zur Herstellung von Verbindungen, dargestellt durch die allgemeine Formel [II] ; RCH(OH)COOH, gemäß Anspruch 8, **dadurch gekennzeichnet, dass** die Verbindung, dargestellt durch die allgemeine Formel [I] ; RCH(OH)CN, worin R wie in Anspruch 1 definiert ist, eine aus der aus Lactonitril, Mandelonitril und 2-Hydroxy-4-methylthiobutyronitril bestehenden Gruppe ausgewählte Verbindung ist.

12. Verfahren zur Herstellung von Verbindungen, dargestellt durch die allgemeine Formel [II] ; RCH(OH)COOH, gemäß Anspruch 8, **dadurch gekennzeichnet, dass** der Mikroorganismus Variovorax paradoxus ist.

13. Verfahren zur Herstellung von Verbindungen, dargestellt durch die allgemeine Formel [II] ; RCH(OH)COOH, gemäß Anspruch 8, **dadurch gekennzeichnet, dass** der Mikroorganismus Arthrobacter Stamm NSSC 104, FERM-BP-5829 ist.

## Revendications

1. Procédé pour préparer des composés répondant à la formule générale [II] RCH(OH)COOH dans laquelle R représente un atome d'hydrogène, un groupe alkyle en C₁-C₆ le cas échéant substitué, un groupe alcényle en C₂-C₆ le cas échéant substitué, un groupe alcoxy en C₁-C₆ le cas échéant substitué, un groupe aryle le cas échéant substitué, un groupe aryloxy le cas échéant substitué ou un groupe hétérocyclique le cas échéant substitué, **caractérisé en ce que** les composés répondant à la formule générale [II] RCH(OH)COOH dans laquelle R est tel que défini ci-dessus sont produits et sont accumulés dans un solvant aqueux en présence d'un micro-organisme possédant à la fois une résistance à la concentration et une propriété de conservation vis-à-vis des composés répondant à la formule générale [I] RCH(OH)CN dans laquelle R est tel que défini ci-dessus et/ou vis-à-vis des composés répondant à la formule générale [II] RCH(OH)COOH dans laquelle R est tel que défini ci-dessus dans le procédé pour transformer un composé répondant à la formule générale [I] RCH(OH)CN dans laquelle R est tel que défini ci-dessus en composés répondant à la formule générale [II] RCH(OH)COOH dans laquelle R est tel que défini ci-dessus, en utilisant une réaction hydrolytique fournie par l'activité enzymatique du micro-organisme, le micro-organisme étant Variovorax paradoxus ou la souche Arthrobacter NSSC 104 déposée sous la dénomination FERM BP-5829.

2. Procédé pour préparer des composés répondant à la formule générale [II] RCH(OH)COOH selon la revendication 1, **caractérisé en ce que** R représente un atome d'hydrogène, un groupe alkyle en C₁-C₆ le cas échéant substitué ou un groupe phényle le cas échéant substitué.

3. Procédé pour préparer des composés répondant à la formule générale [II] RCH(OH)COOH selon la revendication 1 ou 2, **caractérisé en ce que** R représente un atome d'hydrogène, un groupe alkyl(en C₁-C₆)thioalkyle un groupe hydroxyalkyle en C₁-C₆ ou un groupe phényle.

4. Procédé pour préparer des composés répondant à la formule générale [II] RCH(OH)COOH selon la revendication 1, **caractérisé en ce que** le composé répondant à la formule générale [I] RCH(OH)CN est un composé choisi parmi le groupe constitué par le lactonitrile, le mandélonitrile et le 2-hydroxy-4-méthylthiobutyronitrile.

5. Procédé pour préparer des composés répondant à la formule générale [II] RCH(OH)COOH selon l'une quelconque des revendications 1 à 4, **caractérisé en ce que** le micro-organisme est Variovorax paradoxus.

6. Procédé pour préparer des composés répondant à la formule générale [II] RCH(OH)COOH selon l'une quelconque des revendications 1 à 4, **caractérisé en ce que** le micro-organisme est la souche Arthrobacter NSSC 104, FERM BP-5829.

7. Souche Arthrobacter NSSC 104, FERM BP-5829, qui fait partie de l'espèce Arthrobacter, **caractérisée par le fait qu'**elle possède une résistance à la concentration et une propriété de conservation vis-à-vis des composés répondant à la formule générale [I] RCH(OH)CN dans laquelle R représente un atome d'hydrogène, un groupe alkyle en C₁-C₆ le cas échéant substitué, un groupe alcényle en C₂-C₆ le cas échéant substitué, un groupe alcoxy en C₁-C₆ le cas échéant substitué, un groupe aryle le cas échéant substitué, un groupe aryloxy le cas échéant substitué ou un groupe hétérocyclique le cas échéant substitué, et/ou vis-à-vis de composés répondant à la formule générale [II] RCH(OH)COOH dans laquelle R est tel que défini à la revendication 1, et possédant une capacité de transformer un composé répondant à la formule générale [I] RCH(OH)CN en un composé répondant à la formule générale [II] RCH(OH)COOH.

8. Procédé pour préparer des composés répondant à la formule générale [II] RCH(OH)COOH dans laquelle R représente un atome d'hydrogène, un groupe alkyle en C₁-C₆ le cas échéant substitué, un groupe alcényle en C₂-C₆ le cas échéant substitué, un groupe alcoxy en C₁-C₆ le cas échéant substitué, un groupe aryle le cas échéant substitué, un groupe aryloxy le cas échéant substitué ou un groupe hétérocyclique le cas échéant substitué, **caractérisé en ce que**, dans le procédé pour préparer les composés répondant à la formule générale [II] RCH(OH)COOH dans laquelle R est tel que défini à la revendication 1, en se basant sur une hydrolyse d'un composé répondant à la formule générale [II] RCH(OH)COOH dans laquelle R est tel que défini ci-dessus, en utilisant l'activité enzymatique d'un micro-organisme à savoir Variovorax paradoxus ou la souche Arthrobacter NSSC 104, FERM BP-5829, la réaction est mise en oeuvre en présence d'un composé de cyanure répondant à la formule générale [III] Mm(CN)n dans laquelle M représente un atome d'hydrogène, un atome d'ammonium ou un ion métallique, et m et n représentent, indépendamment l'un de l'autre, un entier égal à 1, 2 ou 3.

9. Procédé pour préparer des composés répondant à la formule générale [II] RCH (OH) COOH, selon la revendication 8, **caractérisé en ce que** R représente un atome d'hydrogène, un groupe alkyle en C₁-C₆ le cas échéant substitué ou un groupe phényle le cas échéant substitué.

10. Procédé pour préparer des composés répondant à la formule générale [II] RCH(OH)COOH, selon la revendication 8 ou 9, **caractérisé en ce que** R représente un atome d'hydrogène, un groupe alkyl(en C₁-C₆)thioalkyle, un groupe hydroxyalkyle en C₁-C₆ ou un groupe phényle.

11. Procédé pour préparer des composés répondant à la formule générale [II] RCH(OH)COOH selon la revendication 8, **caractérisé en ce que** le composé répondant à la formule générale [I] RCH(OH)CN dans laquelle R est tel que défini à la revendication 1, est un composé choisi parmi le groupe constitué par le lactonitrile, le mandélonitrile et le 2-hydroxy-4-méthylthiobutyronitrile.

12. Procédé pour préparer des composés répondant à la formule générale [II] RCH(OH)COOH selon la revendication 8, **caractérisé en ce que** le micro-organisme est Variovorax paradoxus.

13. Procédé pour préparer des composés répondant à la formule générale [II] RCH(OH)COOH selon la revendication 8, **caractérisé en ce que** le micro-organisme est la souche Arthrobacter NSSC 104, FERM BP-5829.
